Europäisches Patentamt

European Patent Office

Office européen des brevets

(19)

(11) Publication number: **0 057 763**

**A1**

(12) **EUROPEAN PATENT APPLICATION**

(21) Application number: **81109600.7**

(22) Date of filing: **10.11.81**

(51) Int. Cl.³: **C 07 D 239/91**

(30) Priority: **26.01.81 US 228422**

(43) Date of publication of application:
**18.08.82 Bulletin 82/33**

(84) Designated Contracting States:
**BE CH DE FR GB IT LI NL SE**

(71) Applicant: PENNWALT CORPORATION
Pennwalt Building Three Parkway
Philadelphia Pennsylvania 19102(US)

(72) Inventor: Campanella, Liborio A.
73 Timberline Drive
Penfield New York 14526(US)

(74) Representative: Kraus, Walter, Dr. et al,
Patentanwälte Dres. Kraus & Weisert Irmgardstrasse 15
D-8000 München 71(DE)

(54) Improved process for the preparation of
2-methyl-3-(o-tolyl)-7-chloro-1,2,3,4-tetrahydro-4-oxo-6-quinazolinesulfonamide.

(57) The present invention relates to an improved process for
the preparation of 2-methyl-3-(o-tolyl)-7-chloro-1,2,3,4-
tetrahydro-4-oxo-6-quinazolinesulfonamide by reacting the
corresponding benzamide precursor with acetaldehyde or an
acetal at an elevated temperature in the presence of ethanol
or isopropanol and an acid having a negative pKa.

Croydon Printing Company Ltd.

EP 0 057 763 A1

- 1 -

## Improved Process for the Preparation of 2-Methyl-3-(o-tolyl)-7-Chloro-1,2,3,4-Tetrahydro-4-oxo-6-Quinazolinesulfonamide

### Background of the Invention

The present invention relates to the preparation of 2-methyl-3-(o-tolyl)-7-chloro-1,2,3,4-tetrahydro-4-oxo-6-quinazolinesulfonamide (I) and, more particularly, to an improved process for the final step of the multistage synthesis in which the corresponding benzamide (II) precursor, 2-amino-4-chloro-5-sulfamoyl-N-(o-tolyl) benzamide (II), is reacted with an aldehyde or acetal to obtain ring closure.

United States Patent Nos. 3,360,518; 3,567,746; 3,761,480; and 3,862,949 disclose and claim compound (I) and other 3-aryl-7-halo-1,2,3,4-tetrahydro-4-oxo-6-quinazolinesulfonamide compounds which have been found to be useful as diuretics, and/or methods by which such compounds can be prepared. The specification and claims of these patents are specifically incorporated herein by reference.

According to U. S. Patent No. 3,862,949, compound (II) is prepared by reacting a 5-chloro-2-methyl-aniline with a lower alkyl haloformate to produce an N-carbalkoxy-5-chloro-2-methyl-aniline, reacting said aniline with halosulfonic acid and ammonium hydroxide to produce a N-carbalkoxy-5-chloro-2-methyl-4-sulfamoyl-aniline, reacting said aniline with potassium permanganate to produce N-carbethoxy-5-chloro-4-sulfa-moylanthranilic acid, reacting said anthranilic acid with a cyclizing and condensing agent such as thionyl chloride to produce 7-chloro-6-sulfamoylisatoic anhydride, reacting said isatoic anhydride with o-toluidine to produce 2-amino-4-chloro-5-sulfamoyl-N-(o-tolyl) benzamide (Il) and reacting said benzamide with a suitable cyclizing agent such as an acetal, aldehyde or ketone to produce the desired compound.

The foregoing synthesis route involves 6 basic reactions which are illustrated by equations 1 through 7 on the bottom half of page 4 (columns 5 and 6) and the top half of page 5 (columns 7 and 8) of the printed specifications of U. S. Patent No. 3,862,949. It will of course be obvious to those skilled in the art that these equations illustrate only the 6 basic reactions of the synthesis route, and that each specific reaction may in fact involve more than a single step.

For example, equation No. 6 of U. S. Patent 3,862,949 illustrates reaction of the benzamide (II) with dimethyl acetal to obtain ring closure, the reaction being carried out (column 11, lines 16 seq of U. S. Patent No. 3,862,949) in glacial acetic acid, at ambient temperature, for a period of three and a half hours, after which the crude product was recovered by filtration and washed. In a separate series of crystallization and concentration procedures, the crude product was recrystallized from 95% ethanol to finally obtain the desired product.

Example 34 of U. S. Patent No. 3,761,480 describes preparation of the 2-ethyl-3-(m-tolyl) homologue of compound (I) prepared in the process of the present invention, employing cold ethanol as the solvent. The experiment is reported to have produced only a 47% yield of product.

In repeating the procedure of the preceding experiment in an attempt to synthesize the corresponding 2-methyl-3-(o-tolyl) quinazolinone compound, which is prepared in this invention, there was no product after four hours, and only a trace yield of product after letting the reaction continue overnight.

0057763

- 3 -

## Summary of the Invention

The present invention relates to an improved process for the preparation of compound (I) (as illustrated by equation 6 of U. S. Patent No. 3,862,949) wherein the benzamide precursor (II) is reacted with acetaldehyde or an acetal of the formula $CH_3CH(OR)_2$, wherein R is a hydrocarbon group, to form the desired end product (I).  In the improved process of the present invention, the reaction is carried out at an elevated temperature in the presence of ethanol or isopropanol and of an acid having a negative pKa.

"Acetaldehyde" as used herein includes both acetaldehyde and paraldehyde.  R is preferably a lower alkyl group such as methyl or ethyl.

By "elevated temperature" is meant a temperature of from at least about 40°C to about 85°C.

A stoichiometric excess (normally about 3-50%, preferably about 10-20% of the aldehyde or acetal is usually employed.

The ethanol normally employed is either absolute or an aqueous ethanol of at least 80% concentration, preferably 95%.  About 2.5 to 20 ml. of ethanol per gram of benzamide are normally used, preferably about 2.7 to 9 ml./g.

Similar quantities of isopropanol can be employed, with the isopropanol used normally being absolute isopropanol, although 70% isopropanol is also useful.

The process of the present invention eliminates the acetic acid heretofore employed and also renders optional use of the separate recrystallization step heretofore required.  In addition, it has been found that by employing one of the preferred embodiments of the present invention, the volume of alcohol required as the reaction medium is substantially less than than which was heretofore required merely for recrystallization, and the yield of pure end product (based on the starting benzamide) is significantly higher.

As indicated above, the process of the present invention is carried out at a temperature of from at least about 40°C to the boiling point (reflux temperature) of the alcohol, but preferably at or near the boiling point of the alcohol, normally for a period of from about 0.25 to about five hours, in the presence of an acid having a negative pKa. The molar equivalent of acid to benzamide should be from about 1:10 to 10:1, preferably from 1:4 to 4:1.

The product is recovered by conventional means, such as by cooling the solution and filtering the crystallized product. An additional increase in yield can often be obtained by first concentrating the solution, usually by evaporating a portion of the alcohol.

Preferred Embodiment

In one of the preferred processes of the present invention, the benzamide is suspended in either isopropanol or ethanol. Acid and the aldehyde or acetal are added to the suspension to form a reaction mixture which is then heated and maintained at reflux for a period from about 15 minutes to about four hours, after which the solution is filtered hot and the filtrate concentrated until the volume is from about 10% to about 50% of the original volume of the reaction mixture. Additional aldehyde or acetal is then added (normally about 1-20% stoichiometric equivalent, preferably about 10-12%), and the reaction mixture is refluxed for a further period from about 15 minutes to about one hour, after which the reaction mixture is allowed to cool and the solid product is filtered and washed.

In another preferred embodiment, the benzamide is suspended in either isopropanol or ethanol; the aldehyde or acetal is added; and the reaction mixture is heated to reflux and maintained at reflux, while the acid is added to the hot reaction mixture.

In another preferred embodiment, the benzamide is suspended in isopropanol; the aldehyde is added followed by the acid; and the reaction mixture is heated to reflux for about 1 hr. and then the reaction mixture is allowed to cool and the solid product is filtered and washed.

The following examples will serve by way of illustration and not by way of limitation to describe the process of the present invention and its advantages over the process heretofore employed. As used in the following examples, unless otherwise clearly apparent from the context, ethanol shall be understood to be 95% denatured ethanol and all yields are based on the total amount of 2-amino-4-chloro-5-sulfamoyl-N-(o-tolyl) benzamide starting material.

Prior Art Method

Preparation of 2-methyl-3-(o-tolyl)-7-chloro-1,2,3,4-tetrahydro-4-oxo-6-quinazolinesulfonamide

2-amino-4-chloro-5-sulfamoyl-N-(o-tolyl) benzamide (685 g.) was slurried in 7000 ml. glacial acetic acid. To this slurry was added 240 g. (280 ml.) dimethyl acetal and 4 ml. concentrated sulfuric acid. The reaction mixture was stirred for 3½ hours, then filtered and washed thoroughly with ether. The yield of crude material was 664 g., and it melted at 252-253°C. This was combined with 622 g. of a crude from an identical run and recrystallized from 30 liters of 95% ethanol by dissolving hot, cooling, and concentratingto 12 liters under reduced pressure at 30°C. Weight of the first crop = 1033 g. This solid was recrystallized from 95% ethanol (25 liters) to give a first crop of 685 g. Yield - 46.4% m.p. 246-250°C.

The filtrates from the two recrystallizations were concentrated down to half volume, and a further 251 g. product was obtained. Concentration of this filtrate to half volume gave another 196 g. product which was recrystallized again from 4500 ml. 95% ethanol, by concentration to half volume at 30°C under vacuum, yielding 151 g. of product. This portion was combined with the 251 g. portion obtained above to give a combined wieght of 402 g. Yield - 27.3% m. p. 247-252°C. The total weight of final product was 1087 g. Yield - 73.7%

Example 1

To a suspension of 203.4 g. (0.6M) of 2-amino-4-chloro-5-sulfamoyl-N-(o-tolyl) benzamide 1632 ml. of 95% ethanol was added 29.1 g. (0.22M - a 10% molar excess) of paraldehyde and 60 ml. of concentrated hydrochloric acid. The reaction mixture was brought to reflux and solution occurred within 30 minutes. The hot solution was filtered in portions into a 1 liter round-bottomed flask and concentrated to 540 ml. total volume. The condenser was placed upright, 3.04 g. of paraldehyde (0.023M - a further 15% molar excess of aldehyde) was added and the mixture was refluxed for one hour. The heat was then turned off and the pot temperature was allowed to drift down slowly to 22°C. The solid was then filtered off (actual volume of filtrate 380 ml.) The solid was washed with 2 x 60 ml. portions of cold ethanol. The product, dried at room temperature, weighed 198 g. (90%), m. p. 256-260°c.

Comparing the prior art method described above, with the preferred embodiment illustrated in Example 1, it will be seen that in the prior art method 14 ml. of acetic acid and 60 ml. of ethanol are used to produce 1 gm. of end product, while in the method of Example 1 no acetic acid and less than 10 ml. of ethanol are used per gram of end product. In addition there is 22% improvement in yield, or a 16% increase in yield based on the starting benzamide. An improvement is also seen in Example 7 below 80% yield v. 73.3% in the prior art method) even though the concentration step (which should increase yield) was omitted.

These differences are more clearly apparent from an examination of Table I which sets forth the reagents used per gram of end product for the known prior art method described above and the process of Example 1.

### Table 1

| Reagent | Reagent/g. of Product | |
| | "Prior Art" Process | Example 1 |
| --- | --- | --- |
| 95% ethanol | 33 ml. | 8.8 ml. |
| acetic acid | 6.8 ml. | - |
| chloroform | 2.6 ml. | - |
| methanol | 0.8 ml. | - |
| conc. hydrochloric acid | 0.03 ml. | 0.3 ml. |
| paraldehyde | 0.2 ml. | 0.16 ml. |

Thus, the amount of alcohol in Example 1 is 27% of that used in the prior art process. The paraldehyde is reduced by 20%, and acetic acid, chloroform and methanol are all eliminated. Because concentrated hydrochloric acid is relatively inexpensive, the increased HCl is of no consequence.

The following examples were conducted in a manner similar to that set forth in Example 1.

### Example 2

To 80.0 g. (0.235 mole) of 2-amino-4-chloro-5-sulfamoyl-N-(o-tolyl) benzamide was added 640 ml. ethanol and 25 ml. water, followed by 11.2 g. of acetaldehyde instead of paraldehyde and 6 ml. conc. HCl. The reaction mixture was refluxed for one and a half hours, filtered and the resulting solution was concentrated by distillation to a volume of 160 ml.; 2.6 g. of acetaldehyde was added and the temperature was maintained with stirring for 15 minutes. The product was filtered and washed with 60 ml. hot ethanol instead of cold ethanol. Yield - 70 g. (81%)

Example 3

To 80.0 g. (0.235 mole) of 2-amino-4-chloro-5-sulfamoyl-N-(o-tolyl) benzamide was added 640 ml. ethanol and 25 ml. water, followed by 11.2 g. of paraldehyde and 12 ml. conc. HCl which is twice as much as Example 1 or 2. The reaction mixture was refluxed for one and a half hours and the resulting solution was filtered. The solution was concentrated by distillation to a volume of 230 ml., 1.1 g. paraldehyde was added and it was maintained at reflux with stirring for 15 minutes. The product was filtered and washed with 150 ml. hot ethanol. Yield - 68 g. (80%)

Example 4

To 90.0 g. (0.265 mole) of 2-amino-4-chloro-5-sulfamoyl-N-(o-tolyl) benzamide was added 730 ml. ethanol, followed by 35 g. of 1,1-dimethoxyethane and 30 ml. conc. HCl. The reaction mixture was heated to reflux, and the resulting solution was filtered, concentrated by distillation to a volume of 180 ml., and maintained at reflux with stirring for 15 minutes, then filtered and washed with 100 ml. hot ethanol. Yield - 67 g. (69%)

Example 5

To 20 g. (0.06 M) of 2-amino-4-chloro-5-sulfamoyl-N-(o-tolyl) benzamide was added 160 ml. of absolute ethanol and 6.25 ml. water, followed by 5.7 g. 1,1-dimethoxyethane and a solution of 1.5 ml. conc. sulfuric acid in 4 ml. of water. The reaction mixture was refluxed for one hour, and the near clear solution was filtered. The filtrate was concentrated by distillation to a volume of 50 ml., maintained at temperature for 15 minutes, then filtered and washed with 40 ml. hot 95% ehtanol. Yield - 16.5 g. (77%)

Example 6

To 20 g. (0.06M) of 2-amino-4-chloro-5-sulfamoyl-N-(o-tolyl) benzamide was added 150 ml. of absolute ethanol followed by 5.7 g. of 1,1-dimethoxyethane and 4 ml. of conc. hydrochloric acid. The reaction mixture was refluxed for 30 minutes, filtered, then refluxing was continued during which time solid was formed. The solid which formed after one hour was filtered off, washed with 25 ml. hot 95% ethanol, then dried. Yield - 6.5 g. (31%). Note the short reflux time.

Example 7

To 20.3 g. (0.06M) of 2-amino-4-chloro-5-sulfamoyl-N-(o-tolyl) benzamide was added 100 ml. of absolute ethanol followed by 8.5 g. of 1,1-dimethoxyethane and 20 ml. conc. HCl. The mixture was refluxed, filtered when solution occurred, then refluxing was continued for one and a half hours. Additional solid formed during the course of heating. The reaction was cooled to 15°C, treated with 40 ml. water to precipitate additional product, stirred one half hour, then the solid was filtered off. Yield - 17.5 g. (80%).

Sulfuric acid (pKa -3), hydrochloric acid (pKa -6.11), hydrobromic acid (pKa -9), p-toluenesulfonic acid (pKa -1.34) and methanesulfonic (pKa -1.2) were all successfully employed in the process of the present invention, while phosphoric acid (pKa +2.5), succinic acid (pKa +4.21), and acetic acid (pKa +4.76) did not provide a significant yield of product.

Examples 8 and 9 illustrate another preferred embodiment of the present invention.

Example 8

To 30.6 g. (0.09 mole) of 2-amino-4-chloro-5-sulfamoyl-N-(o-tolyl) benzamide was added 80 ml. of 95% ethanol and 4.5 ml. (0.034 mole) of paraldehyde. The mixture was blanketed with nitrogen and then brought to reflux with stirring. With the pot temperature of 70°C, 9 ml. of conc. hydrochloric acid was added all at once. Complete solution occurred after 2.5 minutes and was maintained for 3 minutes before the product started to crystallize. The mixture was refluxed for 45 minutes and then allowed to cool to room temperature (approx. 1.5 hours). After stirring 3 hours at room temperature, the product was filtered and washed with 25 ml. 95% ethanol. Yield - 30.1 g. (91.4%). On recrystallization from ethanol a 92% yield was obtained thereby resulting in an overall yield of 83.7%.

Example 9

The procedure of Example 8 was repeated but the mixture was only heated to a temperature of about 40°C. A 92% recovery of impure product was obtained; this comprised approximately 20% starting benzamide and 72% quinazolinone end product.

In the embodiment illustrated by Example 8, recrystallization is generally preferred. Even in the embodiment illustrated by Examples 1-7, a recrystallization is often desirable to remove trace impurities or promote recovery of a greater portion of the product in a particular monocrystalline form.

Example 10

To a suspension of 16.3 g. (0.048 m) of 2-amino-4-chloro-5-sulfamoyl-N-(o-tolyl) benzamide in isopropyl alcohol (136 ml) with 3.5 ml (0.026 m) of paraldehyde was added at reflux 9 ml. of concentrated hydrochloric acid over a 2 minute period; solution occurs during the addition. The reaction was maintained at reflux for 15 min.; then the temperature was allowed to drift to 21°C over a 4 hour period. The solid which formed was filtered off, washed with 100 ml. isopropanol, then vacuum dried for 12 hours. The white crystalline product (15.8 g.) represents a 90% recovery of crude. A second crop (1.4 g.) was recovered by concentrating the filtrate. Yield - 17.2 g. (98.0%)

Example 11

To a suspension of 10.0 g. (0.029 m.) of 2-amino-4-chloro-5-sulfamoyl-N-(o-tolyl) benzamide in 70% isopropanol (100 ml.) with 2.0 g. (0.016 m) of paraldehyde was added at reflux 5 ml. of conc. hydrochloric acid. The reaction was kept at reflux for 2 hours with complete solution occurring during the reflux period. The mantle was removed, and the reaction was allowed to drift to 22°C. over 4 hours. The solid was filtered off, isopropanol washed, then air dried. The recovered product (8.7 g.) represents 81% recovery of crude. A second crop (2.0 g.) was recovered on concentrating and cooling the filtrate. Yield - 10.7 g. (99.6%).

Example 12

This is the most preferred method according to this invention.

300 g. (0.88 m) of 2-amino-4-chloro-5-sulfamoyl-N-(o-tolyl) benzamide was added to 785 ml. of isopropanol. The mixture was blanketed with nitrogen, then 49 ml. of paraldehyde was added, followed by 88 ml. of conc. HCl. The reaction is taken to reflux; with a solution resulting within 15 minutes. Reflux is carried on for a total of 45 minutes, then the heating mantle is removed and the mixture is allowed to drift to room temperature (2 hours). Stirring is continued for 5 hours after the removal of the mantle. The solid is then filtered off, washed with 245 ml. isopropanol, then air dried. The yield is 283 g. (88%); m.p. 255-258°C.

The next step was to dissolve 283 g. of crude product in 6257 ml. of hot 95% ethanol which has been denatured with 31 ml. of toluene (6226 ml. 95% ethanol plus 31 ml. toluene). The hot solution and concentrate were filtered keeping the solution hot. It was then added continuously to a 2 liter concentration flask, maintaining it 3/4 full during the concentration. Efficient agitation is maintained to prevent dense crystals from settling to the bottom. 5400 ml. of solvent was distilled followed by the removal of heat to allow the crystals to come to room temperature over a 3 hour period. The product was then filtered off, washed with 300 ml. of denatured 95% ethanol, and air dried. The product weighed 254.4 g. (90%); m.p. 255-256°C.

It will, of course, be obvious to those skilled in the art that many changes and substitutions can be made in the specific materials, reactants and procedural steps set forth hereinbefore, without departing from the scope of the present invention, and it is my intention to be limited only by the appended claims.

WHAT IS CLAIMED IS:

1.    In a process for the preparation of 2-methyl-3-(o-tolyl)-7-chloro-1,2,3,4-tetrahydro-4-oxo-6-quinazolinesulfonamide comprising the reaction of 2-amino-4-chloro-5-sulfamoyl-N-(o-tolyl) benzamide with acetaldehyde, paraldehyde, or an acetal of the formula $CH_3CH(OR)_2$, where R is a hydrocarbon group, the improvement which comprises conducting said reaction at an elevated temperature in the presence of an alcohol selected from ethanol and isopropanol and of an acid having a negative pKa.

2.    The process according to claim 1 wherein said reaction is conducted at the reflux temperature of said alcohol.

3.    The process according to claim 1 wherein said acid is at least one member selected from the group consisting of sulfuric acid, hydrochloric acid, hydrobromic acid, p-toluenesulfonic acid and methanesulfonic acid.

4.    The process according to claim 1 comprising
    a)    suspending said benzamide in said alcohol;
    b)    adding said acid and said acetal or acetaldehyde to said suspension to form a reaction mixture;
    c)    heating said reaction mixture to a temperature of from about 40°C to about 80°C and maintaining said reaction mixture at said temperature for a period from about 15 minutes to about 4 hours;
    d)    recovering said quinazolinesulfonamide.

5.    The process according to claim 4 further comprising, between steps c and d, (i) filtering said reaction mixtures, (ii) concentrating the filtrate to from 10% to 50% of its original volume, (iii) adding to the filtrate from about a 1% to about a 20% stoichiometric equivalent of said acetal or acetaldehyde to form a second reaction mixture at a temperature of from about 40°C to about 80°C for a further period of from about 15 minutes to about one hour.

6. The process according to claim 4 wherein said acid is at least one member selected from the group consisting of sulfuric acid, hydrochloric acid, hydrobromic acid, p-toluenesulfonic acid and methanesulfonic acid.

7. The process according to claim 5 wherein said reactions are conducted at the reflux temperature of said alcohol.

8. The process according to claim 1 comprising
   a) suspending said benzamide in said alcohol;
   b) adding said acetal acetaldehyde to said suspension to form a reaction mixture;
   c) heating said reaction mixture to a temperature of from about 40°C to about 80°C and then adding said acid;
   d) maintaining said reaction mixture at said temperature for a period of from about 15 minutes to about four hours; and
   e) recovering said quinazolinesulfonamide.

9. The process according to claim 8 wherein said acid is at least one member selected from the group cosisting of sulfuric acid, hydrochloric acid, hydrobromic acid, p-toluenesulfonic acid and methanesulfonic acid.

10. The process according to claim 8 wherein said reaction is conducted at the reflux temperature of said reaction.

11.   The process according to claim 10 wherein said alcohol is ethanol.

12.   The process according to claim wherein said alcohol isopropanol.

## European Patent Office

## EUROPEAN SEARCH REPORT

Application number

EP 81 10 9600

### DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | US-A-3 761 480 (BOLA V.SHETTY)<br><br>*Columns 1,11, example 1; column 13, example 7; column 20, example 34; columns 27,28* | 1-7,10,11 | C 07 D 239/91 |
| | --- | | |
| X | GB-A-1 249 396 (WALLACE)<br>*Pages 1,2,10,11* | 1-6 | |
| | --- | | |
| X | GB-A-1 221 407 (WALLACE)<br>*Pages 1,2,15,16* | 1-6 | |
| | --- | | |
| X | CH-A- 515 910 (PERNNWALT)<br>*Pages 1,2,3,12, examples 1,15,16* | 1-6 | |
| | --- | | TECHNICAL FIELDS SEARCHED (Int. Cl. ³) |
| X | US-A-3 793 326 (JAMES F.MUREN)<br>*Column 2 (Va); 4 (XI,XII,Vb); 9 example VIII* | 1,3 | C 07 D 239/00 |
| | --- | | |
| X | FR-A-2 092 058 (R.REIF-WERTHER)<br>*Pages 2,6, example 1, pages 7,11; claims 1,3,4; page 12, claim 5* | 1-11 | |
| | --- | | |
| X | FR-A-2 059 543 (SUMITOMO)<br>*Pages 1,2,3,4, lines 9-10; pages 6,8,9,10* | 1-11 | |
| | ----- | | |

The present search report has been drawn up for all claims

| Place of search | Date of completion of the search | Examiner |
|---|---|---|
| THE HAGUE | 04-05-1982 | FRANCOIS J.C.L. |